# EUROPÄISCHE PATENTANMELDUNG

(11) **EP 4 497 837 A1**
(43) Veröffentlichungstag der Anmeldung: **29.01.2025**
(21) Anmeldenummer: 23188422.2
(22) Anmeldetag: 28.07.2023
(51) Int. Cl.: C12Q 1/689, C12Q 1/70

(54) **VERFAHREN UND ZUSAMMENSETZUNG ZUM IDENTIFIZIEREN VON PHAGEN**

(71) Anmelder: microBIOMix GmbH, 93053 Regensburg (DE)
(72) Erfinder: GESSNER, André, 93053 Regensburg (DE)
(74) Vertreter: Hannke Bittner & Partner mbB Regensburg

(57) **Zusammenfassung**

Die vorliegende Erfindung betrifft ein Verfahren und eine Zusammensetzung zum Identifizieren von Phagen. Das Verfahren umfasst die Schritte:
- Bereitstellen einer Phagenzusammensetzung, welche mindestens zwei verschiedene Phagen umfasst, deren genetischer Code jeweils zumindest abschnittsweise bekannt ist,
- Inkubation einer Bakterienprobe mit der Phagenzusammensetzung,
- Einstellen und Halten der mit der Phagenzusammensetzung inkubierten Bakterienprobe bei Bedingungen bei denen ein Phage ein Bakterium als Wirtszelle nutzen kann,
- Durchführen einer quantitativen Polymerase-Kettenreaktion unter Verwendung von Primerpaaren, die jeweils für einen Phagen spezifisch sind, und
- Identifizierung des und/oder der Phagen, dessen/deren Reproduktion aufgrund der Anwesenheit einer für diese/n Phagen geeigneten Wirtszelle in der Bakterienprobe begünstigt ist.

Außerdem betrifft die Erfindung ein Verfahren zum Quantifizieren von Phagen in einer Probe.

## Beschreibung

Die vorliegende Erfindung betrifft ein Verfahren und Zusammensetzung zum Identifizieren, zur Therapieauswahl (Phagogramm) und zum Quantifizieren (Therapeutisches Phagenmonitoring (TPM)) von Phagen. Eine solche Zusammensetzung wird auch als Kit bezeichnet. Ein solches Kit umfasst bevorzugt mehrere Komponenten, die zur Ausführung des Verfahrens zum Identifizieren von Phagen notwendig sind.

Verschiedene Gruppen von Viren, die auf Bakterien als Wirtszellen spezialisiert sind, werden als Bakteriophagen oder kurz "Phagen" bezeichnet. Im Rahmen dieser Erfindung wird der Begriff "Phagen" verwendet. Da Phagen eine Gruppe der Viren sind, wird auch der Begriff "Viren" verwendet, wobei dieser sich im Rahmen dieser Erfindung auf Viren beschränken soll, die Bakterien befallen. Dementsprechend sollen auch zusammengesetzte Begriffe wie beispielsweise "Virusgenom" als synonym zu "Phagengenom" verstanden werden.

Phagen besitzen keinen eigenen Stoffwechsel und sind für die Vermehrung auf Wirtszellen angewiesen. Die meisten Phagen sind sehr spezifisch auf ein Wirtsbakterium angepasst. Sie weisen daher eine hohe Wirtsspezifität auf. Diese Wirtsspezifität wird auch zu Klassifizierung von Phagen genutzt, zum Beispiel in Coli-, Staphylokokken-, Diphtherie- oder Salmonella-Bakteriophagen.

Mit einer geschätzten Anzahl von 10³⁰ Virionen im gesamten Meerwasser sind Bakterienviren häufiger als jede Art zellulärer Lebewesen. Es kann davon ausgegangen werden, dass nicht alle verschiedenen Phagen bekannt und klassifiziert sind. Darüber hinaus weisen Phagen - ebenso wie Viren - eine große Variabilität auf, so dass ständig Mutationen entstehen können und sich somit neue Phagen (-arten) bilden. Diese Variabilität können Phagen nutzen, um sich schnell an Veränderungen der Wirtszelle anzupassen.

Zur Reproduktion sind Phagen auf eine Bakterienwirtszelle (im Folgenden auch kurz als Wirtszelle bezeichnet) angewiesen. Die Vermehrung vieler Phagen verläuft nach einen lytischen Vermehrungszyklus. Diese Bezeichnung spiegelt wieder, dass die Zellwand der Wirtszelle am Ende des Vermehrungszyklus durch Lyse aufgebrochen und damit die Wirtszelle zerstört wird. In Bakterienkolonien kann der Phagenbefall oftmals durch einen sogenannten Lysehof erkannt werden.

Die Reproduktion von Phagen verläuft meist in fünf Phasen. Zunächst erfolgt eine Adsorption an spezifische Zellwandrezeptoren. Bei dieser Adsorption koppelt ein Phage mit den Enden seiner sogenannten Schwanzfasern an passende Moleküle (Rezeptoren) der Oberfläche des Bakteriums. Die Enden der Schwanzfasern und/oder andere Erkennungssequenzen sind dabei so ausgebildet, dass sie äußerst selektiv eine geeignete Wirtszelle identifizieren und daran koppeln können. Ist eine geeignete Wirtszelle gefunden, erfolgt die Injektion der Phagen-Nukleinsäure in die Wirtszelle. Es wird also die phageneigene Nukleinsäure, (DNA bzw. RNA) in das Bakterium eingebracht. Die nun leere Phagenhülle bleiben meist außerhalb der Zellwand des Bakteriums.

Ist das Phagengenom in die Wirtszelle eingeschleust, beginnt die sogenannte Latenzphase, während der die Transkription des Virusgenoms, die Translation der viralen mRNA und die Replikation der Virusnukleinsäure erfolgt. In der sich daran anschließenden Produktionsphase werden die Phagengene in einer festgelegten Reihenfolge aktiv geschaltet und die Virusbestandteile, Hüllproteine und Schwanzfasern, gebildet. Aus diesen Grundkörpern erfolgt in der Reifephase der Zusammenbau zu Phagenpartikeln. Dabei wird üblicherweise zunächst ein Kopfteil, das sogenannte Kapsid, gebildet, welches die Phagen-Nukleinsäure aufnimmt. Anschließend werden - in Abhängigkeit der Phage - die anderen Komponenten ergänzt.

Sind die Phagenpartikel fertiggestellt, werden diese durch enzymatische Auflösung der Wirtszelle freigesetzt. Dazu ist das Wirtsbakterium derart umprogrammiert worden, dass es Lysozym produziert, welches die bakterielle Mureinzellwand auflöst. Die Zelle platzt und die Phagen werden freigesetzt.

Herkömmlicherweise werden die Bakterienviren (Bakteriophagen) entsprechend ihrer Wirtsspezifität in verschiedene Gruppen klassifiziert, zum Beispiel in Coli-, Staphylokokken-, Diphtherie- oder Salmonella-Bakteriophagen (oder -viren).

Phagen finden heute vielfache Verwendung. Dabei wird insbesondere deren hohe Wirtsspezifität ausgenutzt. So werden beispielsweise Phagen in der Medizin im Rahmen der sogenannten Lysotypie aufgrund ihrer Wirtsspezifität zur Bestimmung von bakteriellen Erregern genutzt. Ebenfalls wurde bereits vor der Entdeckung des Penicillins und der Antibiotika die Möglichkeit der Anwendung von Phagen zur Therapie bakterieller Infektionen entdeckt. Diese Anwendung im Rahmen der sogenannten Phagentherapie gewinnt aufgrund der immer häufiger auftretenden multiplen Antibiotikaresistenzen derzeit an Bedeutung.

Aufgrund der spezifischen Wirkungsweise der Phagen erfolgt die Therapie über speziell hergestellte Phagenzubereitungen. Da Bakteriophagen nicht humanpathogen sind, sondern nur das für sie spezifische Bakterium infiltrieren und abtöten, konnten in allen über 500 bislang veröffentlichten Anwendungsstudien - außer entzündungsspezifischen Symptomen wie vorübergehendem Fieber - keinerlei Nebenwirkungen festgestellt werden. Die Phagentherapie scheint den chemischen Antibiotika nicht nur in Bezug auf Spezifität, sondern auch hinsichtlich Effizienz und Nebenwirkungen überlegen, da herkömmliche Antibiotika zum Teil erhebliche Nebenwirkungen beispielsweise an Leber und Niere aufweisen und einen gravierenden Schaden am menschlichen Mikrobiom anrichten können.

Als Fertigarzneimittel für humanmedizinische Zwecke finden Phagenzubereitungen mit einer Mischung verschiedener Phagen in einigen Ländern des ehemaligen Ostblocks Verwendung. Dort werden derartige Phagenzubereitungen als relativ unspezifisches Breitband-Antibiotikum erfolgreich eingesetzt. Innerhalb der EU gibt es derzeit kein zugelassenes Fertigarzneimittel auf Basis von Phagen. Vereinzelt findet die Phagentherapie jedoch auch in der EU im sogenannten Off-Label-Use und im Rahmen einer Notfall-Indikation Anwendung.

Um Nebenwirkungen zu reduzieren und die Nutzung von relativ unspezifischen Phagenzubereitungen mit mehreren verschiedenen Phagen vermeiden zu können, ist es notwendig, schon vor Beginn einer Phagentherapie ein für den zu behandelnden Patienten personalisiertes und für den pathogenen (gegebenenfalls antibiotikaresistenten) Bakterienstamm spezifisches Phagogramm zu erstellen. Dadurch kann vermieden werden, dass im Rahmen der Phagentherapie ungeeignete Phagen eingesetzt werden (sogenannte Therapieversager). Zur Erstellung dieser Phagogramm fehlt es jedoch an einer geeigneten Infrastruktur (d. h. Phagenlabore). Darüber hinaus sind die derzeit bekannten Verfahren zur Herstellung eines Phagogramms nur beschränkt geeignet. Sie dauern sehr lange und erfordern sowohl eine spezielle Laborausstattung als auch speziell geschultes Personal.

Es besteht daher ein Bedarf an einem Verfahren zur Herstellung eines Phagogramms. Dieses sollte standardisierbar und robust sein. Darüber hinaus sollte es mit üblicher Laborausstattung und Kenntnis durchführbar sein. Außerdem sollte das Phagogramm möglichst schnell vorliegen, vorzugsweise innerhalb von 24 Stunden.

Außerdem besteht Bedarf ein einer Zusammensetzung zum Identifizieren von Phagen. Diese Zusammensetzung sollte leicht handhabbar sein und insbesondere in einem Verfahren zur Herstellung eines Phagogramms nutzbar sein. Möglichst sollte eine solche Zusammensetzung - welche im Folgenden auch als Kit bezeichnet wird - zur Erstellung eines Phagogramms innerhalb von 24 Stunden geeignet sein.

Diese Aufgabe wird durch die Gegenstände der unabhängigen Patentansprüche gelöst. Bevorzugte Ausgestaltungen sind Gegenstand der abhängigen Patentansprüche.

Ein wesentlicher Aspekt der vorliegenden Erfindung ist ein Verfahren zum Identifizieren von Phagen. Dies umfasst die Schritte:
- Bereitstellen einer Phagenzusammensetzung, welche mindestens zwei verschiede-ne Phagen umfasst, deren genetischer Code jeweils zumindest abschnittsweise bekannt ist,
- Inkubation einer Bakterienprobe mit der Phagenzusammensetzung,
- Einstellen und Halten der mit der Phagenzusammensetzung inkubierten Bakterienprobe bei Bedingungen bei denen ein Phage ein Bakterium als Wirtszelle nutzen kann,
- Durchführen einer quantitativen Polymerase-Kettenreaktion unter Verwendung von Primerpaaren, die jeweils für einen Phagen spezifisch sind, und
- Identifizierung des und/oder der Phagen, dessen/deren Reproduktion aufgrund der Anwesenheit einer für diese/n Phagen geeigneten Wirtszelle in der Bakterienprobe begünstigt ist.

Durch ein solches Verfahren kann aus einer Phagenzusammensetzung der und/oder die Phage/n identifiziert werden, welche ein in der Bakterienprobe enthaltenes Bakterium als Wirtszelle nutzen können. Enthält die Bakterienprobe eine für einen Organismus, beispielsweise einen Menschen, insbesondere einen an einer Bakterieninfektion leidenden Organismus und/oder Menschen, schädliche Bakterienart, kann der und/oder die Phage/n als zur Verwendung im Rahmen einer Phagentherapie geeignete/r Phage/n identifiziert werden.

Im Bereich der Virologie besteht im Wesentlichen Einigkeit darüber, dass Viren (und damit auch Bakterienvieren beziehungsweise Phagen nicht zu den Lebewesen zu rechnen sind. Sie werden aber zumeist als "dem Leben nahestehend" betrachtet, da sie allgemein die Fähigkeit zur Evolution sowie zur Steuerung ihrer Replikation haben. Dementsprechend werden Viren üblicherweise auch gemäß der herkömmlichen Taxonomie klassifiziert. Im Rahmen dieser Erfindung werden Phagen, die ein im Wesentlichen identisches Genom aufweisen (also ein genomisch kohärentes Cluster bilden) als eine Phagenart bezeichnet. Der Begriff "Phage" soll - sofern nicht etwas anderes definiert ist - im Rahmen dieser Erfindung als eine Anzahl einzelner Phagen (im Sinn von Virionen) einer einzigen Phagenart verstanden werden.

Die Polymerase-Kettenreaktion (Polymerase Chain Reaction, im Folgenden als PCR bezeichnet) hat sich zu einem wichtigen Instrument in einer Vielzahl von Prozessen entwickelt. Sie wird in vielen Labors routinemäßig eingesetzt. Seit ihrer Entwicklung wurden vielfältige Veränderungen und Weiterentwicklungen vorgenommen. Diese zielen insbesondere auf einen schnellen Temperaturwechsel und auf eine Miniaturisierung und somit auch eine Reduzierung des zu temperierenden Probenvolumens ab. Nachdem die chemischen und biophysikalischen Probleme einer schnellen und miniaturisierten Abarbeitung von diagnostischen PCR-Reaktionen in funktionierende Testsysteme umgesetzt wurden, basieren moderne Geräteplattformen zunehmend auf teil- bzw. vollautomatisierten und physikalisch geschlossenen Testkonzepten.

Von besonderer Bedeutung ist die quantitative Echtzeit-PCR (englisch: real-time quantitative PCR (kurz qPCR oder Q-PCR)). Sie beruht auf dem Prinzip der herkömmlichen Polymerase-Kettenreaktion, erlaubt aber zusätzlich die Quantifizierung der gewonnenen DNA. Die Quantifizierung erfolgt mittels mindestens einem Marker, die ein zu der DNA-Menge korrespondierendes Signal senden können. Oftmals werden als Marker Fluoreszenzsonden eingesetzt, wobei die vorliegende Erfindung nicht auf diese Fluoreszenzsonden beschränkt ist. Die Stärke des zu der DNA-Menge korrespondierenden Signals wird während eines PCR-Zyklus in Echtzeit erfasst, beispielsweise durch Fluoreszenz-Messungen. Auch wenn die vorliegende Erfindung nicht auf die quantitative Echtzeit-PCR zur Quantifizierung der Phagen-DNA beschränkt ist, ist die quantitative Echtzeit-PCR bevorzugt. Andere Methoden zur Quantifizierung der bei der PCR erhaltenen Phagen-DNA (beispielsweise im Anschluss an die PCR) sind jedoch nicht ausgeschlossen.

Vorzugsweise handelt es sich bei der PCR um eine quantitative Multiplex-Real-time-PCR. Dies ermöglicht, eine Vielzahl von Phagen parallel zu quantifizieren. Dies ist insbesondere bevorzugt, da so unmittelbar eine relative Menge der in der Phagenzusammensetzung enthaltenen Phagen ermittelt werden kann. Eine solche relative Menge der in der Phagenzusammensetzung enthaltenen Phagen (beziehungsweise deren Erbgut) ist meist ausreichend, um den Phagen zu ermitteln, welcher sich in einer Probe besonders gut vermehren konnte, da ein für ihn geeignetes Wirtsbakterium in der Probe enthalten war. Die Bestimmung einer absoluten Menge eines Phagen in der Phagenzusammensetzung ist meist nicht notwendig.

Die PCR wird vorzugsweise mit phagenspezifischen Primern durchgeführt. Als phagenspezifischer Primer wird in diesem Zusammenhang ein Primer verstanden, der jeweils komplementär zu einem Abschnitt der DNA eines einzigen in der eingesetzten Phagenzusammensetzung enthaltene Phagen ist. Dadurch kann sichergestellt werden, dass mit einem solchen Primer bzw. Primerpaar nur die DNA eines einzigen Phagen kopiert werden kann.

Wird die PCR als Multiplex-PCR durchgeführt, werden vorzugsweise spezifische und orthogonale Primerpaare eingesetzt. Als orthogonal soll in diesem Zusammenhang ein Primerpaar verstanden werden, welches nicht zum Genom eines anderen (in der Phagenzusammensetzung enthaltenen) Phagen komplementär ist. Durch den Einsatz spezifischer und orthogonaler Primerpaare ist es möglich, Abschnitte des Genoms mehrerer Phagen in einer Phagenzusammensetzung parallel per PCR zu vervielfältigen. Dementsprechend können mehrere Phagen parallel analysiert und/oder quantifiziert werden.

In einer bevorzugten Variante des Verfahrens umfasst die Phagenzusammensetzung 5 - 100, vorzugsweise 7 - 75, bevorzugt 8 - 50, weiter bevorzugt 9 - 40 und insbesondere bevorzugt 10 - 30 verschiedene Phagen. Dadurch kann gleichzeitig eine große Anzahl verschiedener Phagen auf die Eignung zur Nutzung eines in der Bakterienprobe vorhandenen Bakteriums als Wirt zur Reproduktion getestet werden. Dadurch kann wiederum der in der Phagenzusammensetzung enthaltene Phage zur Bekämpfung dieser Bakterien ausgewählt werden. Andererseits hat sich gezeigt, dass aus Phagenzusammensetzungen, die deutlich mehr verschiedenen Phagen enthalten, die zur Bekämpfung des Wirtsbakteriums geeignete Phagenart schwieriger zu identifizieren ist.

Vorzugsweise ist die Sequenz des Genoms jedes in der Phagenzusammensetzung enthaltenen Phagen vollständig bekannt. Dadurch wird die Identifizierung der Phagenvereinfacht. Außerdem ist die Auswahl geeigneter Primer beziehungsweise Primerpaaren möglich, die jeweils speziell für eine einzige Phagenart sind. Dies vereinfacht die Identifizierung und Quantifizierung per Q-PCR.

Bevorzugt ist ein Titer der verschiedenen Phagen in der Phagenzusammensetzung bekannt. Dadurch kann wird die Bewertung der Ergebnisse der Q-PCR und insbesondere die Quantifizerung und der Ausschluss von falsch-positiven-Tests vereinfacht. Vorzugsweise sind die Phagen der Phagenzusammensetzung in etwa mit dem selben Titer enthalten. Vorzugsweise weicht der Titer der verschiedenen Phagen um einen Faktor ≤ 100 %, bevorzugt um einen Faktor ≤ 75 %, weiter bevorzugt um einen Faktor ≤ 50 % und insbesondere bevorzugt um einen Faktor ≤ 25 % voneinander ab.

Als besonders geeignet hat sich das oben beschriebene Verfahren zur Ermittlung von Phagen erwiesen, die eine Bakterienspezies aus der Gruppe *Klebsiella pneumoniae, Pseudomonas aeruginosa, E. faecium* und *Staphylococcus aureus, Staphylococcus epidermidis* als Wirtszelle nutzen.

Daher ist bevorzugt, dass die Bakterienprobe mindestens eine Bakterienspezies aufweist, welche ausgewählt ist aus einer Gruppe, die *Klebsiella pneumoniae, Pseudomonas aeruginosa, E. faecium, Staphylococcus aureus, Staphylococcus epidermidis* und/oder eine multiresistente Bakterienart umfasst und/oder die Bakterienprobe einem Organismus entstammt, der an einer bakteriellen Infektion leidet. Da die zumindest einige Bakterien aus der oben genannten Gruppe oftmals mit bekannten Antibiotika nicht oder zumindest nicht ausreichend effektiv behandelbar sind, ist es somit vergleichsweise einfach möglich, eine zur Zerstörung von Bakterien aus der oben genannten Gruppe geeignete Phagenart zu identifizieren.

Vorzugsweise wird diejenige Phagenart, die ein in der Bakterienprobe enthaltendes Bakterium als Wirts nutzen kann, zur antibakteriellen Behandlung eines Organismus ausgewählt. Als antibakterielle Behandlung eines Organismus soll in diesem Zusammenhang eine Behandlung verstanden werden, die eine Menge der in dem Organismus enthaltenen Bakterien zumindest einer Bakterienart verringert.

Vorzugsweise wird der Fortschritt der antibakteriellen Behandlung eines Organismus zu verschiedenen Zeitpunkten kontrolliert. Dies kann vergleichsweise einfach - da die zur antibakteriellen Behandlung eines Organismus ausgewählten Phagen und insbesondere auch deren Genom bekannt ist - mittels Q-PCR erfolgen. Vorzugsweise erfolgt die nach dem im Folgenden beschriebenen Verfahren:
Verfahren zum Quantifizieren von Phagen in einer Probe, wobei die Probe insbesondere eine Körperflüssigkeit ist oder aus einer Körperflüssigkeit gewonnen wird, umfassend die Schritte:
- Bereitstellen der Probe, wobei die Probe insbesondere eine Körperflüssigkeit ist oder aus einer Körperflüssigkeit gewonnen wird,
- Bereitstellen mindestens eines Primerpaares, welches selektiv für ein Genom einer Phagenart ist, dessen genetischer Code zumindest abschnittsweise bekannt ist,
- Durchführen einer quantitativen Polymerase-Kettenreaktion, und Quantifizierung des und/oder der vorhandenen Phagen der Phagenart in der Probe.

Die derart quantifizierte Phagenart ist vorzugsweise so ausgewählt, dass sie eine Bakterienart als Wirtszelle nutzt, die einen zu überwachenden Organismus befallen hat. Die entkomme Körperflüssigkeit entstammt vorzugsweise einem Organismus, der an einer Infektion dieser Bakterienart leidet.

Vorzugsweise wird die Probe zu verschiedenen Zeitpunkten aus einem zu überwachenden System, insbesondere einem zu überwachenden Organismus, entnommen. Dabei liegen vorzugsweise die verschiedenen Zeitpunkte in einem Zeitraum liegen, der (jeweils unabhängig voneinander) ≥ 1 Stunde und ≤ 3 Monate, bevorzugt ≥ 12 Stunden und ≤ 1 Monat, weiter bevorzugt ≥ 24 Stunden und ≤ 20 Tage, insbesondere bevorzugt ≥ 48 Stunden und ≤ 14 Tage beträgt.

Es hat sich gezeigt, dass eine Bakterieninfektion eines Organismus innerhalb eines wie oben definierten Zeitraums meist abgeklungen oder zumindest stark zurückgegangen ist. Andererseits ist durch die (insbesondere durch die oben als unteren Grenzen erwähnten Zeiten) eine ausreichend genaue Überwachung der Fortschritte der Behandlung überwachbar. Kürzere Intervalle haben sich als nicht sinnvoll erwiesen, da meist die Dauer der Quantifizierung der Phagen per Q-PCR eine ähnliche Zeit in Anspruch nimmt und damit ein Ergebnis der Quantifizierung der Phagen in einer Probe erst nach der Entnahme der folgenden Probe vorliegen würde. Außerdem hat sich gezeigt, dass der Nutzen häufigerer Probenentnahme und der Quantifizierung der darin enthaltenen Phagen meist keinen zusätzlichen Nutzen für den zu überwachenden Organismus bringt, so dass dieser zusätzliche Aufwand meist nicht gerechtfertigt ist. In Ausnahmefällen ist jedoch eine Quantifizierung in kürzeren Zeitintervallen möglich, gegebenenfalls sogar eine Quantifizierung in Echtzeit.

Um den Verlauf der Entwicklung der Menge der Phagen in dem Organismus verfolgen zu können, hat es sich als vorteilhaft gezeigt, dass die verschiedenen Zeitpunkte (zu denen die Proben entnommen werden) jeweils ≤ 10 Tage, bevorzugt ≤ 7 Tage, weiter bevorzugt ≤ 3 Tage, mehr bevorzugt ≤ 1 Tag und insbesondere bevorzugt ≤ 12 Stunden aufeinander folgen. Dadurch ist ein Monitoring des Verlaufs der Phagenmenge möglich (Therapeutisches Phagenmonitoring, TPM). Dementsprechend kann auch detektiert werden, ob die Menge der Phagen in der Probe ansteigt, was auf eine Vermehrung der Phagen in dem System beziehungsweise dem Organismus hinweist und damit auch auf eine Zerstörung derjenigen Bakterien, die von diesen Phagen als Wirtszelle genutzt wurden. Dementsprechend kann auf eine Wirksamkeit der antibakteriellen Behandlung des Organismus mit diesen Phagen geschlossen werden.

Vorzugsweise entstammt die Probe aus einer Quelle, die ausgewählt ist aus einer Gruppe, die Wundsekret, Eiter, bronchoalveolärer Lavage (BAL), Drainage, Blut, Lymphe, Urin, Speichel, Nasensekret, Galle, Pankreassekret, Magensaft und Kot umfasst. Es hat sich gezeigt, dass die Überwachung der Menge der Phagen in einer Probe aus einer dieser Quellen besonders geeignet ist, um den Fortschritt der Behandlung und insbesondere die antibakterielle Wirkung der Phagen zu überwachen.

Ein weiterer wesentlicher Aspekt der vorliegenden Erfindung ist eine Zusammensetzung zum Identifizieren von Phagen, welche in einer Bakterienprobe reproduzierbar sind. Diese Zusammensetzung umfasst mindestens zwei verschiedene Phagen (beziehungsweise Phagenarten), deren genetischer Code jeweils zumindest abschnittsweise bekannt ist, und mindestens je ein Primerpaar, wobei die Primer eines Primerpaars selektiv komplementär zu der DNA-Sequenz des Genoms eines Phagen sind. Eine solche Zusammensetzung ist insbesondere dafür geeignet, in einem wie oben beschriebenen Verfahren eingesetzt zu werden.

Eine solche Zusammensetzung kann auch als "Kit" bezeichnet werden. Als "Kit" werden insbesondere Zusammensetzungen bezeichnet, die - gegebenenfalls sogar von ungeschulten Personen - genutzt werden können, um bestimmte molekularbiologische und/oder (bio-) chemische Reaktionen (gegebenenfalls als Abfolge mehrerer Reaktionen aufeinander) durchzuführen. Dazu enthält ein solches Kit üblicherweise eine Anleitung und gegebenenfalls weitere Reagenzien, die zur Durchführung dieser (Abfolge) molekularbiologischer und/oder (bio-) chemischer Reaktionen notwendig ist.

Vorzugsweise eine Zusammensetzung daher eine Anleitung zur Durchführung eines der wie oben beschriebenen Verfahren.

Vorzugsweise umfasst die Zusammensetzung 5 - 100, vorzugsweise 7 - 75, bevorzugt 8 - 50, weiter bevorzugt 9 - 40 und insbesondere bevorzugt 10 - 30 verschiedene Phagen. Dadurch können mit einer solchen Zusammensetzung gleichzeitig viele verschiedene Phagen auf deren Eignung zur Nutzung eines in einer Probe enthaltenen Bakteriums als Wirt getestet werden.

Vorzugsweise ist jeder Primer jedes Primerpaars komplementär zu einer Sequenz in einem Genom genau eines Phagen in der Zusammensetzung. Dabei ist vorzugsweise bekannt, welches Primerpaar zu dem Genom welches Phagen komplementär ist. Dadurch ist es möglich, bei einer quantitativen PCR (Q-PCR) denjenigen Phagen zu identifizieren, welcher ein in einer Probe enthaltenes Bakterium als Wirt nutzen kann.

Vorzugsweise enthält eine Zusammensetzung dazu mindestens eine Sonde, welche eine Quantifizierung einer bei einer Polymerase-Kettenreaktion mit dem Primerpaar produzierten DNA-Menge ermöglicht. Bei einer solchen Sonde kann es sich beispielsweise um eine sogenannte FRET-Sonde handeln, welche den Förster-Resonanzenergietransfer (FRET) zur Quantifizierung nutzt. Beispiels für derartige FRET-Sonden sind beispielsweise als LightCycler-Sonden, LoopTag-Sonden, TaqMan-Sonden, Molecular Beacons und Scorpion-Primer. Weder das oben beschriebene Verfahren noch die Zusammensetzung ist jedoch auf diese Sonden beschränkt.

Vorzugsweise enthält die Zusammensetzung Phagen in zumindest annähernd ähnlicher Menge. Vorzugsweise weist ein Titer der verschiedenen Phagen in der Zusammensetzung um einen Faktor ≤ 100 %, bevorzugt um einen Faktor ≤ 75 %, weiter bevorzugt um einen Faktor ≤ 50 % und insbesondere bevorzugt um einen Faktor ≤ 25 % voneinander ab. Dies vereinfacht die Quantifizierung per Q-PCR. Insbesondere ist bevorzugt, dass der Titer der einzelnen Phagen bekannt ist.

Weitere Vorteile und Zweckmäßigkeiten sind exemplarisch anhand der nachfolgenden Beschreibung einer bevorzugten Ausführungsform in Verbindung mit den Figuren zu entnehmen. Hierbei zeigen:
- Fig. 1: eine schematische Darstellung eines Ausführungsbeispiels der vorliegenden Erfindung, und
- Fig. 2: eine weitere schematische Darstellung eines Ausführungsbeispiels der vorliegenden Erfindung.

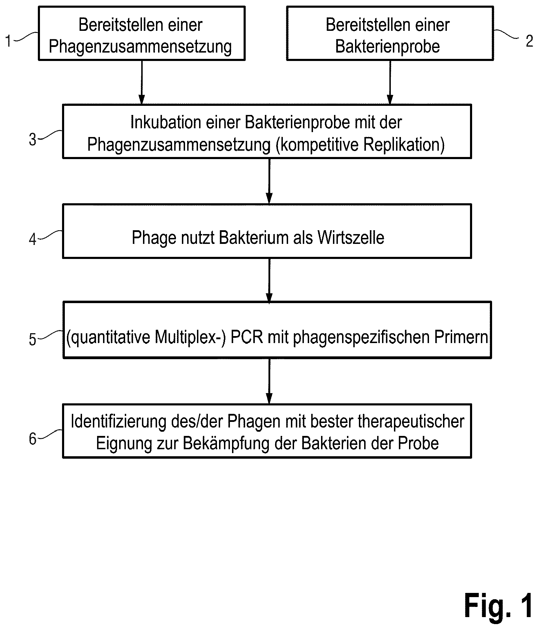

Figur 1 zeigt schematisch den Ablauf eines Verfahrens gemäß der vorliegenden Erfindung. Für dieses Verfahren wird in dem mit Bezugszeichen 1 gekennzeichneten Schritt eine Phagenzusammensetzung bereitgestellt. Diese Phagenzusammensetzung enthält mindestens einen Phagen, von der bekannt ist, welches Bakterium dieser Phage als Wirtszelle dienen kann. Von diesem Phagen ist vorzugsweise auch die DNA-Sequenz zumindest abschnittsweise bekannt.

Vorzugsweise umfasst die Phagenzusammensetzung eine Vielzahl verschiedener Phagen, von denen jeweils bekannt ist, welches Bakterium diesem Phagen als Wirtszelle dienen kann. Vorzugsweise ist von jeder dieser Phagen die DANN-Sequenz bekannt.

In dem mit Bezugszeichen 2 gekennzeichneten Schritt wird eine Bakterienprobe bereitgestellt. Bei dieser Bakterienprobe kann es sich beispielsweise um eine Probe handeln, die aus einer Körperflüssigkeit gewonnen wurde oder die selbst eine Körperflüssigkeit ist.

In dem mit Bezugszeichen 3 kennzeichnenden Schritt wird die Bakterienprobe mit der Phagenzusammensetzung inkubiert. Enthält die Bakterienprobe eine Bakterienart, die einen Wirt für eine der in der Phagenzusammensetzung enthaltenen Phagen darstellt, wird der Phage ein solches Bakterium als potentiellen Wirt erkennen und diesen nutzen.

Wird eine solche mit der Phagenzusammensetzung inkubierte Bakterienprobe unter geeigneten Bedingungen gehalten, wie es schematisch in den mit Bezugszeichen 4 gekennzeichneten Schritt dargestellt ist, kann ein Phage das entspreche Bakterium infizieren und dieses als Wirtszelle nutzen. Nur diejenigen Phagen, für die ein als Wirtszelle geeignetes Bakterium vorhanden ist, werden sich so in der Bakterienprobe vermehren können. Es findet in diesem Schritt folglich eine Vermehrung derjenigen Phagen statt. Andere Phagen werden sich nicht vermehren können oder sogar zerstört bzw. abgebaut werden. Nach einer gewissen Zeit werden daher diejenigen Phagen in der Probe vermehrt vorliegen, für die ein geeignetes Wirtsbakterium in der Probe enthalten ist. Andere Phagen werden sich nicht vermehren oder abgebaut werden.

Wird von dem in Schritt 4 enthaltenen Inkubationsansatz eine Probe für Analysezwecke entnommen, wie es schematisch in dem mit Bezugszeichen 5 beschrifteten Schritt dargestellt ist, kann diese Probe mittels PCR analysiert, insbesondere quantifiziert, werden. Dazu wird die PCR mit phagenspezifischen Primern durchgeführt. Als phagenspezifischer Primer wird in diesem Zusammenhang ein Primer verstanden, der jeweils komplementär zu einem Abschnitt der DNA eines einzigen in der eingesetzten Phagenzusammensetzung enthaltene Phagen ist. Dadurch kann sichergestellt werden, dass mit einem solchen Primer bzw. Primerpaar nur die DNA eines einzigen Phagen kopiert werden kann. Bei der PCR handelt es sich vorzugsweise um eine quantitative PCR. Dabei können mehrere Phagen (beispielsweise durch Zusatz geeigneter spezifischer und orthogonaler (also nicht zum Genom anderer Phagen komplementärer) Primerpaare, parallel analysiert und/oder quantifiziert werden Multipley-PCR). Insbesondere ist somit bevorzugt, die PCR als quantitative Multiplex-Real-time-PCR durchzuführen. Im Zusammenhang mit der Quantifizierung durch PCR wird darauf hingewiesen, dass dabei keine absolute Menge eines (oder mehrerer) Phagengenoms bestimmt werden muss. Vielmehr ist es zur Ermittlung eines für eine Phagentherapie geeigneten Phagen meist ausreichend, eine relative Menge der verschiedenen Phagen in einer Phagenzusammensetzung zu bestimmen.

Ein Primer eines phagenspezifischen Primarpaars enthält vorzugsweise einen Marker, mit dem - vorzugsweise in Echtzeit - der Fortschritt der PCR und damit das Kopieren der DNA des zugehörigen Phagen detektiert werden kann.

Die Detektion der Marker erlaubt dann in einem folgenden Schritt 6 die Identifizierung des oder der Phagen für den/die in der Bakterienprobe ein geeignetes Wirtsbakterium vorhanden war. Aufgrund der eindeutigen Zuordenbarkeit der Primer zu einem Phagen können anhand der Marker derjenige Phage eindeutig identifiziert werden, für den eine geeignete Wirtszelle in der Bakterienprobe vorhanden war. Dementsprechend kann dieser Phage aus der Phagenzusammensetzung ausgewählt werden und dazu genutzt werden, die in der Bakterienprobe enthaltenen Bakterien zu bekämpfen. Dabei ist das Bekämpfen dieser Bakterien nicht auf die Bakterienprobe beschränkt, sondern kann auch in dem Körper erfolgen, aus dem die Bakterienprobe entnommen wurde.

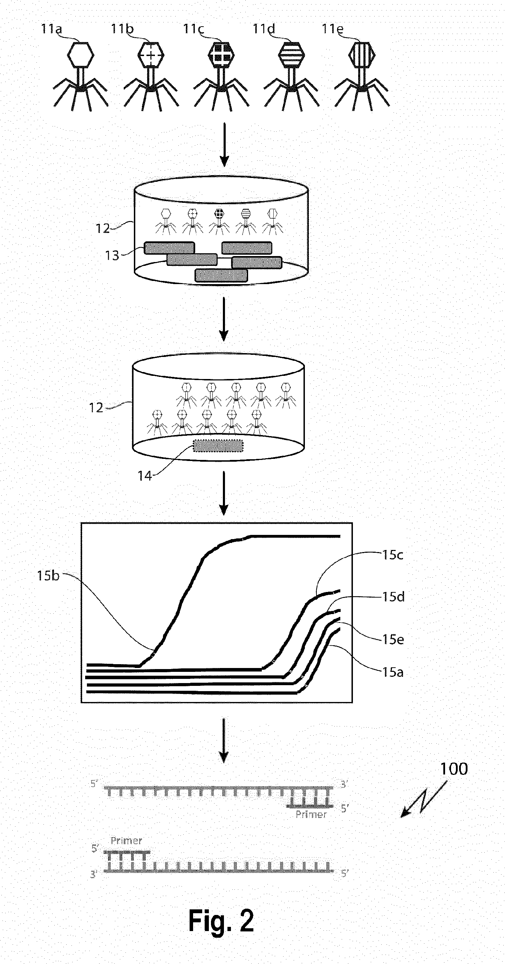

In Figur 2 ist eine weitere schematische Darstellung eines Ausführungsbeispiels der vorliegenden Erfindung gezeigt. Mit den Bezugszeichen 11a -11e sind verschiedene Phagen schematisch dargestellt, die gemeinsam die Phagenzusammensetzung bilden. Von jeder dieser im gezeigten Beispiel fünf Phagen 11a - 11e ist das Genom zumindest abschnittsweise bekannt. Außerdem ist für jede dieser Phagen bekannt, welches Bakterium 13 diesen Phagen als Wirt dienen kann.

Wird eine solche Phagenzusammensetzung in einem geeigneten Reaktor 12 mit einer Probe von Bakterien 13 zusammengebracht, können sich die Phagen vermehren, für die ein geeignetes Wirtsbakterium 13 in der Probe vorhanden ist. Im vorliegenden Beispiel ist dies der mit den Bezugszeichen 11b gekennzeichnete Phage.

Diese Phagen 11b vermehrt sich unter Zerstörung der Bakterien 14, in dem Reaktionsgefäß 12, bis keine geeigneten Wirtszellen mehr vorhanden sind. Andere Phagen, beispielsweise die mit Bezugszeichen 11a, 11c, 11d und 11e gekennzeichneten Phagen, können sich in einer solchen Probe, die lediglich ein einziges Bakterium 13 enthält, welches für den Phagen 11b als Wirtszelle dienen kann, nicht vermehren.

Wird anschließend eine Probe aus dem Reaktionsgefäß 11 entnommen und per PCR analysiert, ist bei Echtzeitquantifizierung erkennbar, dass die exponentielle Phase, also die Phase, in der die Anzahl der bei der PCR kopierten DNA exponentiell zunimmt, für diejenigen Phagen früher anfängt und stärker ausgeprägt ist, die in der Probe in höherer Anzahl vorhanden sind. Im vorliegenden Beispiel ist dies bei dem mit den Bezugszeichen 15b gekennzeichneten Graphen der Fall, welcher für den Phagen 11b steht. Diese Korrelation zwischen dem Graphen 15b und dem Phagen 11b kann dadurch gewährleistet werden, dass dem Marker, welcher zu den Graphen 15b führt, ein Primer (-paar) zugeordnet ist, welcher selektiv eine DNA Sequenz des Phagen 11b bindet.

Bei der PCR wird die exponentielle Phase für diejenigen Graphen später und/oder weniger intensiv ansteigen, deren zugeordnete Phagen 11a, 11c, 11d und 11e in der Probe weniger häufig vorkommen bzw. die sich nicht oder weniger stark vermehren konnten. Dieser spätere exponentielle Anstieg ist für die Graphen 15a, 15c, 15d und 15e dargestellt. Diese Graphen korrelieren jeweils zu einem der mit 11a, 11c, 11d und 11e gekennzeichneten Phagen.

Der Phagen, für den die quantitative PCR das früheste und/oder stärkste exponentielle Wachstum zeigt, wird ausgewählt, um die in der Bakterienprobe enthaltenen Bakterien wirksam bekämpfen zu können. Dieser Phage 11b kann beispielsweise im Rahmen einer Phagentherapie bei einem Patienten eingesetzt werden.

Der Fortschritt der Phagentherapie kann in einem lediglich schematisch mit Bezugszeichen 100 gekennzeichneten Schritt beispielsweise der PCR überwacht werden. Dazu kann analog dem oben dargestellten Schema per quantitativer PCR die DNA des Phagen 11b in einer aus dem Patienten entnommenen Probe quantifiziert werden. Durch einen Vergleich verschiedener PCR Reaktionen, die zu verschiedenen Zeiten nach Beginn der Phagentherapie an aus dem Patienten gewonnenen Proben durchgeführt wurden, lässt sich der Fortschritt der Phagentherapie beobachten. Dabei ist zu erwarten und durch Experimente bestätigt, dass nach dem Beginn einer Phagentherapie eine Menge der in einer aus dem Patienten entnommenen Probe enthaltenen Phagen 11b zunächst ansteigt, da die ausgewählten Fragen mit dem Bakterium 13, das den Patienten infiziert hat, einen geeigneten Wirt 13 vorfinden. Haben sich diese Phagen 11b im Körper des Patienten ausreichend stark vermehrt, gewinnen sie Oberhand gegenüber den Bakterien 13 und sind in so großer Anzahl vorhanden, dass die Menge der Wirtsbakterien 13 im Körper des Patienten verringert wird. Ab diesem Zeitpunkt ist auch die Vermehrung der Phagen 11b verringert, da nicht mehr genügend Wirtszellen 13 zur Vermehrung zur Verfügung stehen. Dieser Zustand kann durch die in Schritt 100 schematisch dargestellte PCR Reaktion überwacht werden.

Der Anmelder behält sich vor, sämtliche in den Anmeldungsunterlagen offenbarten Merkmale als erfindungswesentlich zu beanspruchen, sofern sie einzeln oder in Kombination gegenüber dem Stand der Technik neu sind. Es wird weiterhin darauf hingewiesen, dass in den einzelnen Figuren auch Merkmale beschrieben wurden, welche für sich genommen vorteilhaft sein können. Der Fachmann erkennt unmittelbar, dass ein bestimmtes in einer Figur beschriebenes Merkmal auch ohne die Übernahme weiterer Merkmale aus dieser Figur vorteilhaft sein kann. Ferner erkennt der Fachmann, dass sich auch Vorteile durch eine Kombination mehrerer in einzelnen oder in unterschiedlichen Figuren gezeigter Merkmale ergeben können.

### Bezugszeichenliste

- 1: Schritt, Bereitstellen einer Phagenzusammensetzung
- 2: Schritt, Bereitstellen einer Bakterienprobe
- 3: Schritt, Inkubation der Bakterienprobe mit einer Phagenzusammensetzung
- 4: Schritt, Vermehrung der Phagen
- 5: Schritt, quantitative (Multiplex-Real-time-) PCR
- 6: Schritt, Identifizierung von Phagen mit den besten therapeutischen Eigenschaften

## Patentansprüche

1. Verfahren zum Identifizieren von Phagen umfassend die Schritte:
- Bereitstellen einer Phagenzusammensetzung, welche mindestens zwei verschiedene Phagen umfasst, deren genetischer Code jeweils zumindest abschnittsweise bekannt ist,
- Inkubation einer Bakterienprobe mit der Phagenzusammensetzung,
- Einstellen und Halten der mit der Phagenzusammensetzung inkubierten Bakterienprobe bei Bedingungen bei denen ein Phage ein Bakterium als Wirtszelle nutzen kann,
- Durchführen einer quantitativen Polymerase-Kettenreaktion unter Verwendung von Primerpaaren, die jeweils für einen Phagen spezifisch sind, und
- Identifizierung des und/oder der Phagen, dessen/deren Reproduktion aufgrund der Anwesenheit einer für diese/n Phagen geeigneten Wirtszelle in der Bakterienprobe begünstigt ist.

2. Verfahren nach Anspruch 1,
**dadurch gekennzeichnet, dass**
die Phagenzusammensetzung 5 - 100, vorzugsweise 7 - 75, bevorzugt 8 - 50, weiter bevorzugt 9 - 40 und insbesondere bevorzugt 10 - 30 verschiedene Phagen umfasst.

3. Verfahren nach Anspruch 1 oder 2,
**dadurch gekennzeichnet, dass**
die Sequenz des Genoms jedes in der Phagenzusammensetzung enthaltenen Phagen vollständig bekannt ist.

4. Verfahren nach einem der vorangegangenen Ansprüche,
**dadurch gekennzeichnet, dass**
ein Titer der verschiedenen Phagen in der Phagenzusammensetzung bekannt ist, wobei der Titer der verschiedenen Phagen vorzugsweise um einen Faktor ≤ 100 %, bevorzugt um einen Faktor ≤ 75 %, weiter bevorzugt um einen Faktor ≤ 50 % und insbesondere bevorzugt um einen Faktor ≤ 25 % voneinander abweicht.

5. Verfahren nach einem der vorangegangenen Ansprüche,
**dadurch gekennzeichnet, dass**
die Bakterienprobe mindestens eine Bakterienspezies aufweist, welche ausgewählt ist aus einer Gruppe, die *Klebsiella pneumoniae, Pseudomonas aeruginosa, E. faecium, Staphylococcus aureus* und/oder eine multiresistente Bakterienart umfasst und/oder die Bakterienprobe einem Organismus entstammt, der an einer bakteriellen Infektion leidet.

6. Verfahren zum Quantifizieren von Phagen in einer Probe, wobei die Probe insbesondere eine Körperflüssigkeit ist oder aus einer Körperflüssigkeit gewonnen wird, umfassend die Schritte:
- Bereitstellen der Probe,
- Bereitstellen mindestens eines Primerpaares, welches selektiv für ein Genom einer Phagenart ist, dessen genetischer Code zumindest abschnittsweise bekannt ist,
- Durchführen einer quantitativen Polymerase-Kettenreaktion, und Quantifizierung des und/oder der vorhandenen Phagen der Phagenart in der Probe.

7. Verfahren nach Anspruch 6,
**dadurch gekennzeichnet, dass**
die Probe zu verschiedenen Zeitpunkten aus einem zu überwachenden System, insbesondere einem zu überwachenden Organismus, entnommen wird, wobei vorzugsweise die verschiedenen Zeitpunkte in einem Zeitraum liegen, der ≥ 1 Stunde und ≤ 3 Monate, bevorzugt ≥ 12 Stunden und ≤ 1 Monat, weiter bevorzugt ≥ 24 Stunden und ≤ 20 Tage, insbesondere bevorzugt ≥ 48 Stunden und ≤ 14 Tage beträgt.

8. Verfahren nach Anspruch 7,
**dadurch gekennzeichnet, dass**
verschiedenen Zeitpunkte jeweils ≤ 10 Tage, bevorzugt ≤ 7 Tage, weiter bevorzugt ≤ 3 Tage, mehr bevorzugt ≤ 1 Tag und insbesondere bevorzugt ≤ 12 Stunden aufeinander folgen.

9. Verfahren nach einem der Ansprüche 6 - 8,
**dadurch gekennzeichnet, dass**
die Probe aus einer Quelle stammt, die ausgewählt ist aus einer Gruppe, die Wundsekret, Eiter, bronchoalveolärer Lavage (BAL), Drainage, Blut, Lymphe, Urin, Speichel, Nasensekret, Galle, Pankreassekret, Magensaft und Kot umfasst.

10. Zusammensetzung zum Identifizieren von Phagen, welche in einer Bakterienprobe reproduzierbar sind,
**dadurch gekennzeichnet, dass**
die Zusammensetzung mindestens zwei verschiedene Phagen umfasst, deren genetischer Code jeweils zumindest abschnittsweise bekannt ist, und
mindestens je ein Primerpaar, wobei die Primer eines Primerpaars selektiv komplementär zu der DNA-Sequenz des Genoms eines Phagen sind.

11. Zusammensetzung nach Anspruch 10,
**dadurch gekennzeichnet, dass**
sie 5 - 100, vorzugsweise 7 - 75, bevorzugt 8 - 50, weiter bevorzugt 9 - 40 und insbesondere bevorzugt 10 - 30 verschiedene Phagen umfasst.

12. Zusammensetzung nach einem der Ansprüche 10 oder 11,
**dadurch gekennzeichnet, dass**
das jeder Primer jedes Primerpaars komplementär zu einer Sequenz in einem Genom genau eines Phagen in der Zusammensetzung ist, wobei vorzugsweise bekannt ist, welches Primerpaar zu dem Genom welches Phagen komplementär ist.

13. Zusammensetzung nach einem der Ansprüche 10 - 12,
**gekennzeichnet durch**
mindestens eine Sonde, welche eine Quantifizierung einer bei einer Polymerase-Kettenreaktion mit dem Primerpaar produzierten DNA-Menge ermöglicht.

14. Zusammensetzung nach einem der Ansprüche 10 - 13,
**dadurch gekennzeichnet, dass**
ein Titer der verschiedenen Phagen in der Zusammensetzung um einen Faktor ≤ 100 %, bevorzugt um einen Faktor ≤ 75 %, weiter bevorzugt um einen Faktor ≤ 50 % und insbesondere bevorzugt um einen Faktor ≤ 25 % voneinander abweicht.

15. Zusammensetzung nach einem der Ansprüche 10 - 14,
**dadurch gekennzeichnet, dass**
sie eine Anleitung zur Durchführung eines Verfahrens nach einem der Ansprüche 1 - 5 und/oder der Ansprüche 6 - 9 umfasst.
